# EUROPEAN PATENT SPECIFICATION

(11) **EP 3 586 960 B1**
(45) Date of publication and mention of the grant of the patent: **26.01.2022**
(21) Application number: 17897284.0
(22) Date of filing: 27.02.2017
(51) Int. Cl.: B01J 29/85, C01B 37/08, C01B 39/54, C07C 1/20, B01D 53/94, B01J 35/00, B01J 35/02

(54) **CU-SAPO MOLECULAR SIEVE, SYNTHESIS METHOD THEREFOR AND CATALYTIC USE THEREOF**
CU-SAPO-MOLEKULARSIEB, SYNTHESEVERFAHREN DAFÜR UND KATALYTISCHE VERWENDUNG DAVON
TAMIS MOLÉCULAIRE CU-SAPO, SON PROCÉDÉ DE SYNTHÈSE ET SON UTILISATION CATALYTIQUE

(43) Date of publication of application: 01.01.2020
(73) Proprietor: Dalian Institute Of Chemical Physics, Chinese Academy of Sciences, Liaoning 116023 (CN)
(72) Inventor: TIAN, Peng, Dalian Liaoning 116023 (CN); XIANG, Xiao, Dalian Liaoning 116023 (CN); LIU, Zhongmin, Dalian Liaoning 116023 (CN); CAO, Lei, Dalian Liaoning 116023 (CN)
(74) Representative: Witte, Weller & Partner Patentanwälte mbB
(86) International application number: PCT/CN2017/074985
(87) International publication number: WO 2018/152829

(56) References cited:
- EP-A1- 2 269 733
- WO-A1-2011/112949
- WO-A1-2016/154391
- CN-A- 101 932 384
- CN-A- 106 268 935
- US-A1- 2016 243 531
- ALESSANDRO TURRINA ET AL: "Retrosynthetic Co-Templating Method for the Preparation of Silicoaluminophosphate Molecular Sieves", CHEMISTRY OF MATERIALS, vol. 28, no. 14, 26 July 2016 (2016-07-26) , pages 4998-5012, XP055382885, US ISSN: 0897-4756, DOI: 10.1021/acs.chemmater.6b01676
- PLEVERT ET AL: "Faulting effects in the CHA-GME group of ABC-6 materials", PROCEEDINGS OF THE INTERNATIONAL ZEOLITE CONFERENCE, 12TH, BALTIMORE, JULY 5-10, 1998,, 1 January 1998 (1998-01-01), pages 2445-2452, XP009502867, ISBN: 978-1-55899-463-8
- SKEELS G W ET AL: "Synthesis and characterization of phi-type zeolites LZ-276 and LZ-277: faulted members of the ABC-D6R family of zeolites", MICROPOROUS AND MESOPOROUS MATERIALS, ELSEVIER, AMSTERDAM, NL, vol. 30, no. 2-3, 1 September 1999 (1999-09-01), pages 335-346, XP004174879, ISSN: 1387-1811, DOI: 10.1016/S1387-1811(99)00045-1

## Description

### TECHNICAL FIELD

The present invention relates to a novel copper-containing SAPO molecular sieve, a synthesis process therefor and a use thereof in denitration reaction.

### BACKGROUND

NOx, as one of the main atmospheric pollutants, can cause many environmental problems such as acid rain and photochemical smog, and poses a serious hazard to human health. Nitrogen oxide pollution is mainly caused by the emission of tail gas of a mobile source vehicle and the emission of waste gas of stationary source. Selective catalytic reduction of NOx by NH₃, urea or hydrocarbon as a reducing agent is the main method to control the pollution, and NOx is converted into harmless nitrogen. The traditional denitration catalyst is mainly V-Ti-W catalytic system. However, with the widespread adoption of lean-burn technology in engine technology, the emission temperature of the lean-burn exhaust gas is lowered, and the application range of narrower temperature of the catalyst of the V-Ti-W system cannot meet the requirements. Furthermore the potential for the V-W-Ti catalyst to pollute the environment also limits its application. Molecular sieve catalytic system has gradually become a research hotspot. Among the catalysts of the molecular sieve system, the copper-based catalyst and the iron-based catalyst are two representative systems. The copper-based catalysts exhibit excellent low-temperature activity, but an excessively high load causes a severe NH₃ oxidation reaction in the high temperature section. The iron-based catalysts have excellent high temperature activity, but their lower conversion rate in the low temperature range limits their application in certain fields.

In 1986, Iwamoto et al. reported for the first time that Cu²⁺ exchanged ZSM-5 has the capability of catalyzing NO to directly decompose into N₂ and O₂. In following studies, researchers focused on SCR (Selective Catalytic Reduction) reaction with hydrocarbon as reducing agent, Fe-ZSM-5 has gradually become a next research hotspot. Compared with the oxide catalyst, the catalyst of the molecular sieve system has the advantages of wider reaction temperature window, good thermal stability and strong sulfur poisoning resistance at a high temperature, but they also have some problems such as poor high-temperature hydrothermal stability, poor low-temperature sulfur resistance and the like.

Small pore molecular sieves such as SSZ-13 and SAPO-34 as carrier materials, can effectively improve the high-temperature hydrothermal stability of the catalyst, and when loaded with copper as the active metal, it has high NO conversion activity and high N₂ selectivity within a wide temperature range. Although there are some problems such as sensitivity to sulfur, this problem has gradually been solved with the improvement of oil quality.

Generally, the synthesis of SAPO molecular sieves requires organic amine/ammonium as a structure directing agent, which is synthesized by hydrothermal or solvothermal methods. Innovations in synthetic methods and the selection of templating agents have a critical impact on the control of the structure and performance of the product. Cu-SAPO-18 and Cu-SAPO-34 can be synthesized in one-step by using a copper amine complex as a template. Cu-SAPO catalyst synthesized by the one-step process simplifies the preparation process of the catalyst and is of great significance. Moreover, Cu-SAPO type catalyst synthesized by this one-step process shows excellent NH₃-SCR catalytic activity, and tunable denaturation of composition, and has a certain industrial application prospect.

The present invention provides a one-step synthesis method of Cu-SAPO molecular sieve catalyst with controlled Cu content, which shows excellent deNOx catalytic activity, and has potential application value.

WO2011/112949 A1 discloses disordered molecular sieve supports for the selective catalytic reduction of NOx. Examples are directed to copper-containing CHA/AEI intergrown molecular sieves.

Alessandro Turrina et al. (2016), Chemistry of Materials, vol. 28, No. 14, pp. 4998-5012, discloses a Retrosynthetic Co-Templating Method for the Preparation of Si licoaluminophosphate Molecular Sieves.Plevert et al. (1998), Proceedings of the International Zeolite Conference, 12th, Baltimore, pp. 2445-2452, discloses a co-templated method to prepare molecular sieves of the ABC-6 group, such as the silicoaluminophosphate versions of AFX (SAPO-56), SFW (STA-18), and GME (STA-19) topology types.

Skeels G. W. et al. (1999), Microporous and Mesoporous Materials, vol. 30, No. 2-3, pp. 335-346, is directed to the synthesis and characterization of phi-type zeolites LZ-276 and LZ-277, faulted members of ABC-D6R family of zeolites.

US 2016/243531A1 discloses processes for preparing zincoaluminosilicates with AEI, CHA, and GME topologies and compositions derived therefrom.

EP 2269733 A1 discloses a process for the direct synthesis of Cu- containing silicoaluminophosphate (Cu-SAPO-34), and its use in SCR.

### SUMMARY OF THE INVENTION

It is an object of the present invention to provide a Cu-SAPO molecular sieve with eutectic structure of GME and CHA.

The novel molecular sieve synthesized by the present invention exhibits the characteristics of coexistence of broad peaks and narrow peaks, and its XRD diffraction patterns are similar to the spectra of silicon-aluminum zeolite with GME/CHA coexisting structure described in literature (Microporous and Mesoporous Materials, 30 (1999) 335-346*;* the official website of the International Zeolite Association is *http:*//*www.iza-structure.org*/*databases*/*Catalog*/*ABC_6.pdf*). It is analyzed that this type of material was a novel SAPO molecular sieve with a GME/CHA coexisting structure.

According to an embodiment of the present invention, there is provided a Cu-SAPO molecular sieve with coexisting crystal phases of CHA and GME, characterized in that the X-ray diffraction pattern of the molecular sieve comprises at least the following diffraction peaks:

**Table 1**

| No. | **2θ** | **FWHM [2θ] (half-peak width)** |
|---|---|---|
| 1 | 7.40~7.56 | 0.15~0.35 |
| 2 | 9.20~9.70 | 0.25~0.65 |
| 3 | 12.80~12.92 | 0.06~0.09 |
| 4 | 15.80~16.21 | 0.15~0.40 |
| 5 | 17.60~17.82 | 0.05~0.11 |
| 6 | 20.30~20.90 | 0.15~0.40 |
| 7 | 21.75~22.05 | 0.06~0.09 |
| 8 | 25.65~26.05 | 0.05~0.09 |
| 9 | 30.11~30.96 | 0.20~0.45 |

An inorganic framework of the molecular sieve has the following chemical composition: wCu-(SiₓAl_{y}P_{z})O₂, wherein x, y and z respectively represent the molar fractions of Si, Al and P, the molar fraction ranges thereof are respectively x=0.01~0.28, y=0.35~0.55 and z=0.28~0.50 with x+y+z=1, w is the molar number of Cu per mole of (SiₓAl_{y}P_{z})O₂, and w=0.001~0.124.

The anhydrous chemical composition of the molecular sieve comprising a templating agent is expressed as wCu·mR1·nR2·(SiₓAl_{y}P_{z})O₂, wherein R1 is diisopropanolamine or diethanolamine, R2 is trimethylamine; m is the molar number of templating agent R1 per mole of (SiₓAl_{y}P_{z})O₂, n is the molar number of templating agent R2 per mole of (SiₓAl_{y}P_{z})O₂, m=0.01~0.20, n=0.01~0.10; x, y and z respectively represent the molar fractions of Si, Al and P, and the the molar fraction ranges thereof are respectively x=0.01~0.28, y=0.35~0.55 and z=0.28~0.50 with x+y+z=1; w is the molar number of Cu per mole of (SiₓAl_{y}P_{z})O₂, w = 0.001~0.124. In some embodiments, m can be 0.02-0.15; n can be 0.01~0.09; x can be 0.05~0.28; y can be 0.40~0.50; z can be 0.30~0.50; and w can be 0.005~0.100.

It is another object of the present invention to provide a method for synthesizing the above Cu-SAPO molecular sieve, which comprises the steps of:
a) mixing a copper source, deionized water, templating agents R1 and R2, a silicon source, an aluminum source and a phosphorus source in proportion to obtain an initial gel mixture having the following molar ratios:
   Cu/Al₂O₃ = 0.01~ 0.25;
   SiO₂/Al₂O₃ = 0.05~2.0;
   P₂O₅/Al₂O₃ = 0.5~1.5;
   H₂O/Al₂O₃ = 8~40;
   R1/Al₂O₃=5~20;
   R2/Al₂O₃=0.1~1.5;
   wherein R1 is diisopropanolamine (DIPA) or diethanolamine (DEOA); R2 is any one of trimethylamine (TMA), benzyltrimethylammonium chloride (BTACl), benzyltrimethylammonium hydroxide (BTAOH) or a mixture thereof;
   optionally, an order of specific mixing process can be as follows: firstly mixing the copper source with water, then adding R1 and R2, stirring at room temperature for 0.5~5 hours, then adding the aluminum source, the silicon source and the phosphorus source to the mixed solution, and stirring the mixed gel at room temperature for 1~5 hours;
b) feeding the initial gel mixture obtained in step a) into a high pressure synthesis kettle, closing the kettle, heating to 160~ 220 °C and performing crystallization for 5 to 72 hours; and
c) after the crystallization is completed, separating a solid product, washing and drying to obtain the molecular sieve.

Wherein in the step a), the silicon source is selected from one or more of silica sol, activated silica, orthosilicate and metakaolin; the aluminum source is selected from one or more of aluminum salt, activated alumina, pseudoboehmite, alkoxy aluminum and metakaolin; the phosphorus source is selected from one or more of orthophosphoric acid, ammonium hydrogen

It is still another object of the present invention to provide a catalyst for selective catalytic reduction of NOₓ by NH₃, which is obtained by calcining the molecular sieve synthesized according to the above method at 550- 700 °C in air. The benefits brought out by the present invention include:
(1) a novel Cu-SAPO molecular sieve is provided.
(2) the prepared molecular sieve can be used as a catalyst for catalytic removal of nitrogen oxides and exhibits good catalytic performance.

### BRIEF DESCRIPTION OF THE DRAWINGS

FIG. 1 is a XRD pattern of a synthesized product in Example 1.
FIG. 2 is a scanning electron micrograph (SEM) of a synthetic product in Example 1.
FIG. 3 is an evaluation result of NH₃-SCR reaction of Example 11 and Comparative Example 2.
FIG. 4 is an evaluation result of NH₃-SCR reaction of catalysts with different copper contents (Examples 11-13).
FIG. 5 is a comparison of the evaluation results of NH₃-SCR reaction before and after high-temperature hydrothermal treatment for the sample of Example 1 (Example 11 and Example 14)
FIG. 6 is a XRD result of samples corresponding to Comparative Examples 3-8.

### DETAILED DESCRIPTION OF THE EMBODIMENT

The present invention will be futher described in combination with examples. It shoulde be understood that these examples are merely illustrative of the present application and are not to limit the scope of the present invention, which is defined by the appended claims.

The experimental methods which do not specify the specific conditions in the following examples are generally carried out according to conventional conditions or according to the conditions recommended by the manufacturer. Unless otherwise specified, the raw materials used in this application are purchased commercially and directly used without special treatment.

Unless otherwise specified, the test conditions used in the present application were as follows:
Elemental composition was measured using Magix 2424 X-ray fluorescence analyzer (XRF) from Philips.

FT-IR is collected using German BRUKER TENSOR 27 instrument.

X-ray powder diffraction phase analysis (XRD) was carried out on an X'Pert PRO X-ray diffractometer of PANalytical Corporation, Netherlands, using a Cu target, a **Kα radiation source (λ = 0.15418 nm), a voltage of 40 KV** and a current of 40 mA.

The specific surface area and pore size distribution of the samples were determined using a Micromeritics ASAP Model 2020 physical adsorber. Before the analysis, the sample was vacuumized and heated at 350 °C for pretreating for 6 h, and the free volume of the sample tube was measured with He as the medium. When the sample was analyzed, the physical adsorption and desorption measurements were carried out at a liquid nitrogen temperature (77 K) using nitrogen as an adsorption gas. The specific surface area of the material was determined using the BET formula; the total pore volume of the material was calculated using the amount of adsorption of N₂ at a relative pressure (P/P₀) of 0.99. The micropore surface area and micropore volume were calculated by the *t*-plot method. When calculating, the cross-sectional area of the N₂ molecule is 0.162 nm².

SEM morphology analysis was performed using a Hitachi (SU8020) type scanning electron microscope.

Infinity plus 400WB solid nuclear magnetic resonance spectrum analyzer of Varian Corporation, U.S., is used in carbon nuclear magnetic resonance (¹³C MAS NMR) analys, with a BBO MAS probe and an operatinal magnetic field strength of 9.4T.

CHN elemental analysis was performed using a German-made Vario EL Cube elemental analyzer.

The invention is described in detail below by means of examples, but the present invention is not limited to these examples.

### EXAMPLE 1

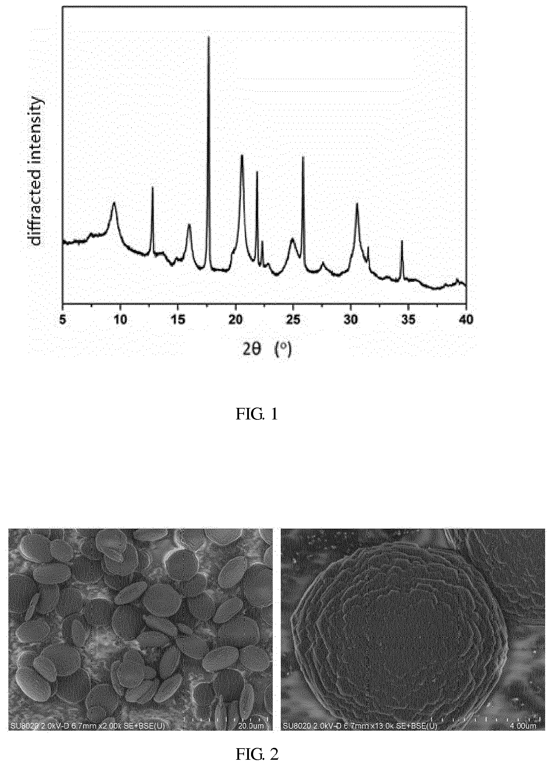

The molar ratio of each raw material and the crystallization conditions were shown in Table 2. The specific mixing process was as follows: firstly the copper source was mixed and dissolved with water, then R1 and R2 were added, and stirred at room temperature for 2 hours. Subsequently, an aluminum source, a silicon source and a phosphorus source were sequentially added to the mixed solution, and the mixed gel was stirred at room temperature for 5 hours to form a gel. The gel was transferred to a stainless steel reactor. After the reactor was placed in an oven, it was heated at a rate of 2 °C/min to 200 °C for crystallization under rotation for 36 hours. After the crystallization was completed, the solid product was centrifuged, washed and dried in air at 100 °C to obtain a sample of the molecular sieve raw powder. The sample was subjected to XRD analysis, and the peak shape exhibited characteristics of broad peaks and narrow peaks. The XRD diffraction pattern was shown in FIG. 1, and the XRD diffraction data was shown in Table 3. After the sample was calcined and the template was removed, the specific surface area and pore volume are measured. The sample had a high BET specific surface area of 602 m²g⁻¹ and a large pore volume of 0.27 cm³g⁻¹, in which the specific pore surface area and micropore volume calculated according to the *t*-plot method were 533 m²g⁻¹ and 0.26 cm³g⁻¹ respectively.

The scanning electron micrograph of the obtained sample was shown in FIG. 2. It can be seen that the morphology of the obtained sample was in a circular sheet shape which was layered and stacked, and the particle size range was 3-5 µm.

**Table 2: Molecular sieve synthesis ingredients and crystallization conditions**

| **Exam ple No.** | **Type and molar amount of R1** | **Type and molar amount of R2** | **Copper source and mole number of CuO contained** | **Aluminum source and mole number of Al₂O₃ contained** | **Phosphorus source and mole number of P₂O₅ contained** | **Silicon source and mole number of SiO₂ contai ned** | **H₂ O** | **Crystallization temperature** | **Crystallization time** |
|---|---|---|---|---|---|---|---|---|---|
| 1 | diethanolamine 0.6 mol | trimethylamine 0.03 mol | 0.009molCuCl₂ | pseudoboeh mite 0.1mol | orthophosphoric acid 0.08mol | Silica sol 0.08mol | 1.5 mol | 200°C | 36h |
| 2 | diethanolamine 0.5 mol | benzyltrimethylammonium chloride 0.01 mol | 0.009mo1CuS O₄ | pseudoboeh mite 0.1mol | ammonium hydrogen phosphate 0.05 mol | Silica sol 0.015m ol | 0.8 mol | 170°C | 48h |
| 3 | diisopropanol amine 0.8 mol | trimethylamine 0.12 mol | 0.006molCu( NO₃)₂ | pseudoboeh mite 0.1mol | orthophosphoric acid 0.10mol | Activat ed silica 0.08mol | 1.1 mol | 220°C | 5h |
| 4 | diethanolamin e 1.2 mol | benzyltrimethylam monium chloride 0.095 mol | 0.009molCu( OAc)₂ | γ - alumina 0.10mol | ammonium dihydrogen phosphate 0.15mol | Silica sol 0.06mol | 2.2 mol | 170°C | 52h |
| 5 | diethanolamin e 0.6 mol | trimethylamine 0.015 mol | 0.009molCuC | pseudoboeh mite 0.1mol | ammonium dihydrogen phosphate 0.12mol | Silica sol 0.02mol | 1.8 mol | 180°C | 50h |
| 6 | diethanolamin e 0.8 mol | trimethylamine 0.06 mol | 0.009molCuC | pseudoboehmite 0.1 mol | phosphoric anhydride 0.13mol | Activat ed silica 0.06mol | 1.5 mol | 170°C | 40h |
| 7 | diethanolamine 1.0 mol | benzyltrimethylammonium hyderoxide 0.075mol | 0.009molCuC | γ - alumina 0.1mol | orthophosphoric acid 0.10mol | Silica sol 0.03mo l | 2.0 mol | 165°C | 60h |
| 8 | diethanolamine 0.6 mol | trimethylamine 0.03 mol | 0.025molCuCl₂ | pseudoboehmite 0.1mol | orthophosphoric acid 0.08mol | Silica sol 0.08mol | 1.5 mol | 200°C | 36h |
| 9 | diethanolamine 0.6 mol | trimethylamine 0.03mol | | pseudoboehmite 0.1mol | orthophosphoric acid 0.08mol | Silica sol 0.08mol | 1.5 mol | 200°C | 36h |

**Table 3: XRD results of the sample in Example 1**

| No. | **2θ** | **d(Å)** | **FWHM [2θ]** | **100xI/I⁰** |
|---|---|---|---|---|
| 1 | 7.5332 | 11.73461 | 0.306 | 3.78 |
| 2 | 9.4392 | 9.36932 | 0.3328 | 16.7 |
| 3 | 12.8276 | 6.90145 | 0.0769 | 30.02 |
| 4 | 13.5764 | 6.52213 | 0.613 | 2.41 |
| 5 | 14.9902 | 5.91019 | 0.301 | 1.97 |
| 6 | 15.9732 | 5.54833 | 0.33542 | 17.36 |
| 7 | 17.6521 | 5.0242 | 0.0895 | 100 |
| 8 | 19.7388 | 4.49821 | 0.1534 | 8.02 |
| 9 | 20.5465 | 4.32274 | 0.2042 | 50.11 |
| 10 | 21.8947 | 4.05968 | 0.0769 | 43.13 |
| 11 | 22.3319 | 3.98041 | 0.0515 | 13.91 |
| 12 | 22.8456 | 3.89277 | 0.2047 | 4.33 |
| 13 | 24.9568 | 3.56792 | 0.4603 | 15.56 |
| 14 | 25.852 | 3.44654 | 0.067 | 51.61 |
| 15 | 27.5942 | 3.23261 | 0.2556 | 6.68 |
| 16 | 30.5503 | 2.92623 | 0.2305 | 33.11 |
| 17 | 31.4892 | 2.83871 | 0.0628 | 15.19 |
| 18 | 33.192 | 2.69914 | 0.4088 | 3.1 |
| 19 | 34.4342 | 2.60441 | 0.0519 | 19.26 |
| 20 | 35.583 | 2.52287 | 0.5116 | 2.9 |
| 21 | 39.3457 | 2.29004 | 0.634 | 2.01 |
| 22 | 42.6011 | 2.12223 | 0.0765 | 11.02 |
| 23 | 43.3283 | 2.08657 | 0.072 | 8.95 |
| 24 | 47.5951 | 1.90894 | 0.0932 | 10.18 |
| 25 | 48.6442 | 1.87181 | 0.4093 | 3.11 |
| 26 | 50.5224 | 1.80502 | 0.0936 | 20.97 |
| 27 | 50.6481 | 1.80533 | 0.075 | 11.88 |
| 28 | 53.192 | 1.72051 | 0.1096 | 9.48 |
| 29 | 54.151 | 1.69223 | 0.072 | 8.1 |
| 30 | 54.9008 | 1.67102 | 0.076 | 11.21 |
| 31 | 55.9254 | 1.64276 | 0.2498 | 2.42 |
| 32 | 59.4243 | 1.55415 | 0.9988 | 1.11 |
| 33 | 60.9672 | 1.51848 | 0.2493 | 2.01 |
| 34 | 61.6477 | 1.50332 | 0.2497 | 2.63 |
| 35 | 62.6378 | 1.48193 | 0.3744 | 1.15 |

### EXAMPLE 2

The specific proportion of ingredients and crystallization conditions were shown in Table 2, and the specific mixing process was the same as that of Example 1.

The synthesized samples were subjected to XRD analysis, and representative data results were shown in Table 4.

Scanning electron micrographs showed that the morphology of the obtained sample was similar to that of the sample of Example 1.

**Table 4: XRD results of the sample of Example 2**

| **No.** | **2θ** | **FWHM [2θ] (half-peak width)** |
|---|---|---|
| 1 | 7.403 | 0.156 |
| 2 | 9.207 | 0.632 |
| 3 | 12.801 | 0.072 |
| 4 | 15.807 | 0.381 |
| 5 | 17.601 | 0.052 |
| 6 | 20.302 | 0.388 |
| 7 | 21.754 | 0.084 |
| 8 | 25.688 | 0.085 |
| 9 | 30.1143 | 0.434 |

### EXAMPLE 3

The specific proportion of ingredients and crystallization conditions were shown in Table 2, and the specific mixing process was the same as that of Example 1.

The synthesized samples were subjected to XRD analysis, and representative data results were shown in Table 5.

Scanning electron micrographs showed that the morphology of the obtained sample was similar to that of the sample of Example 1.

**Table 5: XRD results of the sample in Example 3**

| **No.** | **2θ** | **FWHM [2θ] (half-peak width)** |
|---|---|---|
| 1 | 7.556 | 0.331 |
| 2 | 9.623 | 0.256 |
| 3 | 12.911 | 0.068 |
| 4 | 16.203 | 0.157 |
| 5 | 17.812 | 0.102 |
| 6 | 20.884 | 0.153 |
| 7 | 22.015 | 0.075 |
| 8 | 26.031 | 0.074 |
| 9 | 30.941 | 0.207 |

### EXAMPLES 4-9

The specific proportion of ingredients and crystallization conditions were shown in Table 2, and the specific mixing process was the same as in Example 1.

The synthesized samples were subjected to XRD analysis. The results of XRD data of Examples 4 and 9 were similar to those of Table 3. The results of XRD data of Examples 5 and 6 were similar to those of Table 4, and the results of XRD data of Examples 7 and 8 were close to Table 5.

By comparing with the diffraction spectrum of the different proportions of GME/CHA symbiotic silicon-aluminum zeolite crystal phase given on the official website of the International Zeolite Association, the content of CHA crystal phase in the silicon-phosphorus-aluminum molecular sieve provided in Examples 1-9 was significantly higher than that of GEM crystal phase.

### EXAMPLE 10

¹³C MAS NMR analysis of the powder samples of Examples 1-9 was carried out. Compared with ¹³C MAS NMR standard spectrum of diisopropanolamine, diethanolamine and trimethylamine, it was found that the samples synthesized by using diisopropanolamine as a solvent had resonance peaks of diisopropanolamine and trimethylamine, and the samples synthesized by using diethanolamine as a solvent had resonance peaks of diethanolamine and trimethylamine. Quantitative analysis was performed based on the specific non-coincident NMR peaks of the two substances to determine the ratio of the two substances.

Bulk elemental composition of the molecular sieve product was performed by XRF analysis, and CHN eletmental analysis was also conducted on the raw powder of Examples 1-9. The composition of the molecular sieve raw powder obtained by comprehensive CHN elemental analysis, XRF and ¹³C MAS NMR analysis, was shown in Table 6.

**Table 6: Composition of the raw powder samples in Examples 1-9**

| Example | Composition of the raw powder samples |
|---|---|
| 1 | 0.038Cu0.07DEOA0.02TMA(Si_{0.203}Al_{0.470}P_{0.327})O₂ |
| 2 | 0.008Cu0.04DEOA0.01TMA(Si_{0.032}Al_{0.490}P_{0.478})O₂ |
| 3 | 0.029Cu0.081DIPA0.058TMA(Si_{0.205}Al_{0.488}P_{0.307})O₂ |
| 4 | 0.040Cu0.13DEOA0.048TMA(Si_{0.165}Al_{0.488}P_{0.347})O₂ |
| 5 | 0.018Cu0.07DEOA0.02TMA(Si_{0.072}Al_{0.470}P_{0.458})O₂ |
| 6 | 0.035Cu0.11DEOA0.034TMA(Si_{0.155}Al_{0.488}P_{0.357})O₂ |
| 7 | 0.020Cu0.12DEOA0.038TMA(Si_{0.100}Al_{0.488}P_{0.412})O₂ |
| 8 | 0.09Cu0.07DEOA0.02TMA(Si_{0.203}Al_{0.470}P_{0.327})O₂ |
| 9 | 0.07DEOA0.02TMA(Si_{0.203}Al_{0.470}P_{0.327})O₂ |

The raw powder samples of Examples 1-9 were separately ground and pressed with potassium bromide and subjected to FT-IR characterization. They all showed a characteristic vibration absorption peak attributed to the double six-membered ring at 637 cm⁻¹, showing the presence of a double six-membered ring in the sample.

### EXAMPLE 11

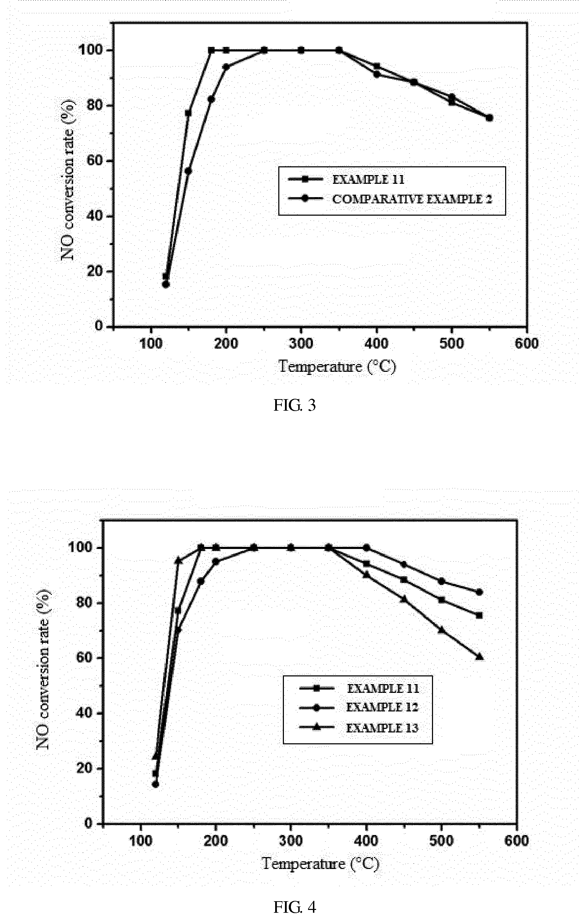

The sample obtained in Example 1 was calcined at a high temperature of 650 °C for 2 hours. After the templating agent was removed, the catalytic performance for the selective catalytic reduction of NOₓ by NH₃ was evaluated. The specific experimental procedures and conditions were as follows: after calcination, the sample was tableted and sieved. 0.1 g of 60-80 mesh of the sample was weighed and mixed with 0.4 g of quartz (60-80 mesh), and charged into a fixed bed reactor. The catalysts was purged by nitrogen at 600 °C for 40 min, then the temperature was lowered to 120 °C to start the evaluation, and the temperature was raised to 550 °C step by step. The component of the raw gas was: NO: 500 ppm, NH₃: 500 ppm, O₂: 5%, H₂O: 5%, N₂ as a balance gas, and a gas flow rate of 300 mL/min. The reaction tail gas was analyzed by online FTIR using a Bruker Tensor 27 instrument. The results were shown in FIG. 3 and FIG. 4. It can be seen that the conversion of NO was 77% at 150 °C, and the conversion of NO was higher than 90% over a wide temperature range of 180-450 °C. Similarly, the samples obtained in Examples 2-8 exhibited excellent catalytic performance for selective reduction of NOₓ after the same treatment as that of the sample of Example 1.

### EXAMPLE 12

The sample obtained in Example 3 was calcined at a high temperature of 650 °C for 2 hours. After the templating agent was removed, the catalytic performance for the NH₃ selective reduction of NOₓ reaction was characterized. The specific experimental procedures and conditions were as follows: after calcination, the sample was tableted and sieved. 0.1 g of 60-80 mesh of the sample was weighed and mixed with 0.4 g of quartz (60-80 mesh), and charged into a fixed bed reactor. The catalysts was purged by nitrogen at 600 °C for 40 min, then the temperature was lowered to 120 °C to start the evaluation, and the temperature was raised to 550 °C step by step. The component of the raw gas was: NO: 500 ppm, NH₃: 500 ppm, O₂: 5%, H₂O: 5%, N₂ as a balance gas, and a gas flow rate of 300 mL/min. The reaction tail gas was analyzed by online FTIR using a Bruker Tensor 27 instrument. The reaction results were shown in FIG. 4.

### EXAMPLE 13

The sample obtained in Example 8 was calcined at a high temperature of 650 °C for 2 hours. After the templating agent was removed, the catalytic performance for the NH₃ selective reduction of NOₓ reaction was characterized. The specific experimental procedures and conditions were as follows: after calcination, the sample was tableted and sieved. 0.1 g of 60-80 mesh of the sample was weighed and mixed with 0.4 g of quartz (60-80 mesh), and charged into a fixed bed reactor. The catalysts was purged by nitrogen at 600 °C for 40 min, then the temperature was lowered to 120 °C to start the evaluation, and the temperature was raised to 550 °C step by step. The component of the raw gas was: NO: 500 ppm, NH₃: 500 ppm, O₂: 5%, H₂O: 5%, N₂ as a balance gas, and a gas flow rate of 300 mL/min. The reaction tail gas was analyzed by online FTIR using a Bruker Tensor 27 instrument. The reaction results were shown in FIG. 4.

### EXAMPLE 14

The sample obtained in Example 1 was calcined at a high temperature of 650 °C for 2 hours. After the templating agent was removed, hydrothermal aging treatment was performed at 800 °C, the content of water vapor was 100%, and the treatment time was 24 hours. It was dried at 100 °C after the treatment.

The relative crystallinity of the sample was determined by XRD method, and the crystallinity of the sample was 95% compared with Example 1, indicating that the sample prepared in Example 1 had high hydrothermal stability, the integrity of the structure of the sample can be well maintained after water treatment.

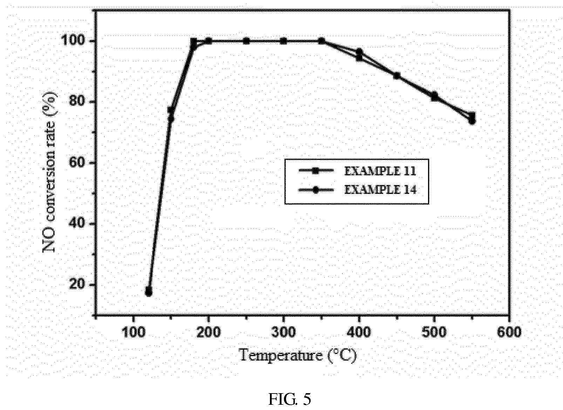

The catalytic performance for NH₃ selective reduction of NOₓ reaction was characterized. The specific experimental procedures and conditions were as follows: after calcination, the sample was tableted and sieved. 0.1 g of 60-80 mesh of the sample was weighed and mixed with 0.4 g of quartz (60-80 mesh), and charged into a fixed bed reactor. The catalysts was purged by nitrogen at 600 °C for 40 min, then the temperature was lowered to 120 °C to start the evaluation, and the temperature was raised to 550 °C step by step. The component of the raw gas was: NO: 500 ppm, NH₃: 500 ppm, O₂: 5%, H₂O: 5%, N₂ as a balance gas, and a gas flow rate of 300 mL/min. The reaction tail gas was analyzed by online FTIR using a Bruker Tensor 27 instrument. The reaction results were shown in FIG. 5.

### COMPARATIVE EXAMPLE 1

10 g of the sample molecular sieve raw powder obtained in Example 9 was used as a precursor, and was heated at a rate of 2 °C/min to a constant temperature of 600 °C for 4 hours to remove the organic templating agent and water contained therein.

The calcined sample was put into a 3.66 mol/L ammonium nitrate aqueous solution at a solid-liquid ratio (mass ratio) of 1:10, stirred for 5 minutes, and then heated to 80 °C for ion exchange for 2 hours. After being centrifugaed and washed for three times with deionized water and dried at 80 °C, a NH4⁺ molecular sieve was obtained.

7 g of NH4⁺ molecular sieve was put into a 0.03 mol/L Cu(CH₃COO)₂·H₂O solution at a solid-liquid ratio of 1:25. After being stirred for 5 minutes, the mixture was heated to 50 °C to ion exchange for 4 hours. After being centrifugaed and washed for three times with deionized water and dried at 80 °C, an obtained sample was denoted as Cu-9/T. The XRF elemental analysis showed that the product had a copper oxide content of 3.2%, which was close to that of Example 1. The specific surface area and pore volume of the calcined type Example 9 and the Cu-9/T sample were measured by N₂ physical adsorption, and the specific pore surface area and pore volume were calculated by the *t*-plot method. The micropore specific surface area and micropore volume of the sample of Example 9 were 559 m²g⁻¹ and 0.28 cm³g⁻¹, respectively. The micropore specific surface area and micropore volume of the Cu-9/T sample were 520 m²g⁻¹ and 0.25 cm³g⁻¹, respectively. These results show that the catalyst prepared according to the method of Example 1 can better maintain the regularity of framework structure of the sample.

### COMPARATIVE EXAMPLE 2

The sample obtained in Comparative Example 1 was calcined at a high temperature of 650 °C for 2 hours, and used as a catalyst for NH₃ selective reduction of NOₓ reaction. The specific experimental procedures and conditions were as follows: after calcination, the sample was tableted and sieved. 0.1 g of 60-80 mesh of the sample was weighed and mixed with 0.4 g of quartz (60-80 mesh), and charged into a fixed bed reactor. The catalysts was purgedby nitrogen at 600 °C for 40 min, then the temperature was lowered to 120 °C to start the evaluation, and the temperature was raised to 550 °C step by step. The component of the raw gas was: NO: 500 ppm, NH₃: 500 ppm, O₂: 5%, H₂O: 5%, N₂ as a balance gas, and the total gas flow rate was 300 mL/min. The total space velocity of the reaction was GHSV = 180000 h⁻¹. The reaction tail gas was analyzed by online FTIR using a Bruker Tensor 27 instrument. The specific results were shown in FIG. 3.

### COMPARATIVE EXAMPLE 3

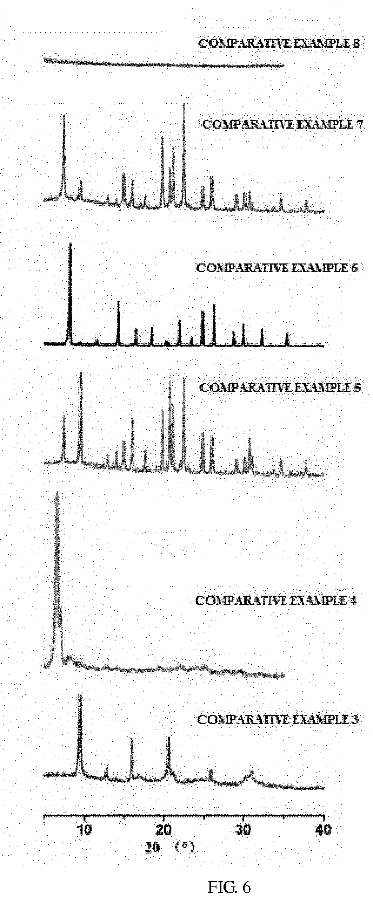

The molar ratio of the specific ingredients, the raw materials and the crystallization conditions were the same as that of Example 1, except that the diethanolamine in the raw material was replaced with triethylamine. The synthesized sample was SAPO-34 molecular sieve, and the XRD analysis results were shown in FIG. 6.

### COMPARATIVE EXAMPLE 4

The molar ratio of the specific ingredients, the raw materials and the crystallization conditions were the same as that of Example 2, except that the benzyltrimethylammonium hyderoxide in the raw material was replaced with 1,6-hexanediamine. The synthesized sample was a lamellar phase, and the XRD results were shown in FIG. 6.

### COMPARATIVE EXAMPLE 5

The molar ratio of the specific ingredients, the raw materials and the crystallization conditions were the same as that of Example 3, except that the addition of trimethylamine in the raw materials was omitted. The synthesized sample was a physical mixture of SAPO-34 and SAPO-5, and the XRD results were shown in FIG. 6.

### COMPARATIVE EXAMPLE 6

The molar ratio of the specific ingredients, the raw materials and the crystallization conditions were the same as that of Example 4, except that the diethanolamine in the raw material was replaced with diethylamine. The synthesized sample was a physical mixture of DNL-6 (SAPO molecular sieve with RHO structure) with a small amount of SAPO-34, and the XRD results were shown in FIG. 6.

### COMPARATIVE EXAMPLE 7

The molar ratio of the specific ingredients, the raw materials and the crystallization conditions were the same as as that of Example 5, except that the trimethylamine in the raw material was replaced with triethanolamine. The synthesized sample was a physical mixture of SAPO-5 and SAPO-34, and the XRD results were shown in FIG. 6.

### COMPARATIVE EXAMPLE 8

The molar ratio of the specific ingredients, the raw materials and the crystallization conditions were the same as that of Example 5, except that the addition of trimethylamine in the raw materials was omitted. The synthesized sample was amorphous, and the XRD analysis results were shown in FIG. 6.

The results of the synthesis of Comparative Examples 3-8 showed that the Cu-SAPO molecular sieve with coexisting crystal phases of CHA and GME of the present patent application can be obtained only under a specific combination of templating agents and material was replaced with triethanolamine. The synthesized sample was a physical mixture of SAPO-5 and SAPO-34, and the XRD results were shown in FIG. 6.

### COMPARATIVE EXAMPLE 8

The molar ratio of the specific ingredients, the raw materials and the crystallization conditions were the same as that of Example 5, except that the addition of trimethylamine in the raw materials was omitted. The synthesized sample was amorphous, and the XRD analysis results were shown in FIG. 6.

The results of the synthesis of Comparative Examples 3-8 showed that the Cu-SAPO molecular sieve with coexisting crystal phases of CHA and GME of the present patent application can be obtained only under a specific combination of templating agents and suitable crystallization conditions.

While the present application has been described above with reference to preferred embodiments, these embodiments are not intended to limit the protection scope of the present invention, which is defined by the appended claims.

Without departing from the scope of the present invention, people skilled in the art will be able to make several possible variations and modifications.

## Claims

1. A copper-containing silicoaluminophosphate (SAPO) molecular sieve with coexisting crystal phases of CHA and GME, wherein the X-ray diffraction pattern of the molecular sieve comprises at least the following diffraction peaks:
| **No.** | **2θ** | **FWHM [2θ] (half-peak width)** |
|---|---|---|
| 1 | 7.40~7.56 | 0.15~0.35 |
| 2 | 9.20~9.70 | 0.25~0.65 |
| 3 | 12.80~12.92 | 0.06~0.09 |
| 4 | 15.80~16.21 | 0.15~0.40 |
| 5 | 17.60~17.82 | 0.05~0.11 |
| 6 | 20.30~20.90 | 0.15~0.40 |
| 7 | 21.75~22.05 | 0.06~0.09 |
| 8 | 25.65~26.05 | 0.05~0.09 |
| 9 | 30.11~30.96 | 0.20~0.45 |

2. The molecular sieve of claim 1, wherein an inorganic framework of the molecular sieve has the following chemical composition: wCu-(SiₓAl_{y}P_{z})O₂, wherein x, y and z respectively represent the molar fractions of Si, Al and P, the molar fraction ranges thereof are respectively x=0.01~0.28, y=0.35~0.55 and z=0.28~0.50 with x+y+z=1, w is the molar number of Cu per mole of (SiₓAl_{y}P_{z})O₂, and w=0.001~0.124.

3. The molecular sieve of claim 2, wherein the anhydrous chemical composition of the molecular sieve comprising a templating agent is expressed as wCu·mR1·nR3·(SiₓAl_{y}P_{z})O₂, wherein R1 is diisopropanolamine or diethanolamine, R2 is trimethylamine; m is the molar number of templating agent R1 per mole of (SiₓAl_{y}P_{z})O₂, n is the molar number of templating agent R2 per mole of (SiₓAl_{y}P_{z})O₂, m=0.01~0.20, n=0.01~0.10; x, y and z respectively represent the molar fractions of Si, Al and P, and the the molar fraction ranges thereof are respectively x=0.01~0.28, y=0.35~0.55 and z=0.28~0.50 with x+y+z=1; w is the molar number of Cu per mole of (SiₓAl_{y}P_{z})O₂, w = 0.001~0.124.

4. A method for synthesizing the molecular sieve of any one of claims 1-3, comprising the steps of:
a) mixing a copper source, deionized water, templating agents R1 and R2, a silicon source, an aluminum source and a phosphorus source in proportion to obtain an initial gel mixture having the following molar ratios:
Cu/Al₂O₃ = 0.01~ 0.25;
SiO₂/Al₂O₃ = 0.05~2.0;
P₂O₅/Al₂O₃ = 0.5~1.5;
H₂O/Al₂O₃ = 8~40;
R1/Al₂O₃₌5~20;
R2/Al₂O₃₌0.1~1.5;
wherein R1 is diisopropanolamine (DIPA) or diethanolamine (DEOA); R2 is any one of trimethylamine (TMA), benzyltrimethylammonium chloride (BTACl), benzyltrimethylammonium hydroxide (BTAOH) or a mixture thereof;
b) feeding the initial gel mixture obtained in step a) into a high pressure synthesis kettle, closing the kettle, heating to 160~220 °C and performing crystallization for 5 to 72 hours; and
c) after the crystallization is completed, separating a solid product, washing and drying to obtain the molecular sieve.

5. The method of claim 4, wherein in the step a), a mixing process is as follows: firstly mixing the copper source with water, then adding R1 and R2, stirring at room temperature for 0.5~5 hours, then adding the aluminum source, the silicon source and the phosphorus source to the mixed solution, and stirring the mixed gel at room temperature for 1~5 hours.

6. The method of claim 4, wherein in the step a), the silicon source is selected from one or more of silica sol, activated silica, orthosilicate and metakaolin; the aluminum source is selected from one or more of aluminum salt, activated alumina, pseudoboehmite, alkoxy aluminum and metakaolin; the phosphorus source is selected from one or more of orthophosphoric acid, ammonium hydrogen phosphate, ammonium dihydrogen phosphate, organic phosphorus compounds and phosphoric oxides; the copper source is selected from one or more of Cu(OAc)₂, CuSO₄, Cu(NO₃)₂ and CuCl₂.

7. The method of claim 4, wherein in the step b), the crystallization is performed in a static or dynamic state.

8. The method of claim 4, wherein in the step a), the initial gel mixture has R1/Al₂O₃ = 5. 0~10.

9. The method of claim 4, wherein in the step a), the initial gel mixture has R2/Al₂O₃ = 0.25~1.0.

10. A catalyst for NOₓ selective catalytic reduction, which is obtained by calcining the molecular sieve synthesized according to the method of any one of claims 4 to 9 at 550~700 °C in air.

## Patentansprüche

1. Kupferhaltiges Silicoaluminophosphat (SAPO) Molekularsieb mit koexistierenden Kristallphasen von CHA und GME, wobei das Röntgenbeugungsmuster des Molekularsiebs mindestens die folgenden Beugungspeaks aufweist:
| **Nr.** | **2θ** | **FWHM [2θ] (Halbwertsbreite)** |
|---|---|---|
| 1 | 7,40 ~ 7,56 | 0,15 ~ 0,35 |
| 2 | 9,20 ~ 9,70 | 0,25 ~ 0,65 |
| 3 | 12,80 ~ 12,92 | 0,06 ~ 0,09 |
| 4 | 15,80 ~ 16,21 | 0,15 ~ 0,40 |
| 5 | 17,60 ~ 17,82 | 0,05 ~ 0,11 |
| 6 | 20,30 ~ 20,90 | 0,15 ~ 0,40 |
| 7 | 21,75 ~ 22,05 | 0,06 ~ 0,09 |
| 8 | 25,65 ~ 26,05 | 0,05 ~ 0,09 |
| 9 | 30,11 ~ 30,96 | 0,20 ~ 0,45 |

2. Molekularsieb nach Anspruch 1, wobei ein anorganisches Gerüst des Molekularsiebs die folgende chemische Zusammensetzung aufweist: wCu-(SiₓAl_{y}P_{z})O₂, wobei x, y und z jeweils die molaren Anteile von Si, Al und P darstellen, wobei die molaren Anteilsbereiche davon x = 0,01 - 0,28, y = 0,35 - 0,55 und z = 0,28 - 0,50 mit x + y + z = 1 sind, wobei w die Molzahl von Cu pro Mol (SiₓAl_{y}P_{z})O₂ ist, und wobei w = 0.001 - 0,124 ist.

3. Molekularsieb nach Anspruch 2, wobei die wasserfreie chemische Zusammensetzung des Molekularsiebs, die ein Templateagens aufweist, als wCu · mR1 · nR3 · (SiₓAl_{y}P_{z})O₂ ausgedrückt wird, wobei R1 Diisopropanolamin oder Diethanolamin ist, R2 Trimethylamin ist; m die molare Anzahl des Templateagens R1 pro Mol (SiₓAl_{y}P_{z})O₂ ist, n die molare Anzahl des Templateagens R2 pro Mol (SiₓAl_{y}P_{z})O₂ ist, m = 0,01 ~ 0,20, n = 0,01 ~ 0,10; x, y und z jeweils die molaren Anteile von Si, Al und P darstellen, und die molaren Anteilsbereiche davon jeweils x = 0,01 ~ 0,28, y = 0,35 ~ 0,55 und z = 0,28 ~ 0,50 mit x + y + z = 1 sind; wobei w die molare Anzahl von Cu pro Mol (SiₓAl_{y}P_{z})O₂ ist, und wobei w = 0,001 ~ 0,124 ist.

4. Verfahren zur Synthese des Molekularsiebs nach einem der Ansprüche 1 bis 3, das die folgenden Schritte aufweist:
a) Mischen einer Kupferquelle, von entionisiertem Wasser, Templateagenzien R1 und R2, einer Siliciumquelle, einer Aluminiumquelle und einer Phosphorquelle in einem Verhältnis, um eine anfängliche Gelmischung zu erhalten, die folgende molare Verhältnisse aufweist:
Cu/Al₂O₃ = 0,01 ~ 0,25;
SiO₂/Al₂O₃ = 0,05 ~ 2,0;
P₂O₅/Al₂O₃ = 0,5 ~ 1,5;
H₂O/Al₂O₃ = 8 ~ 40;
R1/Al₂O₃=5 ~ 20;
R2/Al₂O₃=0,1 ~ 1,5;
worin R1 Diisopropanolamin (DIPA) oder Diethanolamin (DEOA) ist; R2 eines von Trimethylamin (TMA), Benzyltrimethylammoniumchlorid (BTACl),Benzyltrimethyl-ammoniumhydroxid (BTAOH) oder eine Mischung davon ist;
b) Einspeisen der in Schritt a) erhaltenen anfänglichen Gelmischung in einen Hochdruck-Synthesekessel, Schließen des Kessels, Erhitzen auf 160 ~ 220 °C und Durchführen der Kristallisation für 5 bis 72 Stunden; und
c) nach Abschluss der Kristallisation Abtrennung eines festen Produkts, Waschen und Trocknen, um das Molekularsieb zu erhalten.

5. Verfahren nach Anspruch 4, wobei in Schritt a) ein Mischprozess wie folgt abläuft:
zunächst Mischen der Kupferquelle mit Wasser, dann Zugabe von R1 und R2, Rühren bei Raumtemperatur für 0,5 ~ 5 Stunden, dann Zugabe der Aluminiumquelle, der Siliziumquelle und der Phosphorquelle zu der gemischten Lösung und Rühren des gemischten Gels bei Raumtemperatur für 1 ~ 5 Stunden.

6. Verfahren nach Anspruch 4, wobei in Schritt a) die Siliziumquelle ausgewählt ist aus einem oder mehreren von Kieselsol, aktiviertem Siliziumdioxid, Orthosilikat und Metakaolin; die Aluminiumquelle ausgewählt ist aus einem oder mehreren von Aluminiumsalz, aktiviertem Aluminiumoxid, Pseudoböhmit, Alkoxyaluminium und Metakaolin; die Phosphorquelle ausgewählt ist aus einem oder mehreren von Orthophosphorsäure, Ammoniumhydrogenphosphat, Ammoniumdihydrogenphosphat, organischen Phosphorverbindungen und Phosphoroxiden; die Kupferquelle ausgewählt ist aus einem oder mehreren von Cu(OAc)₂, CuSO₄, Cu(NO₃)₂ und CuCl₂.

7. Verfahren nach Anspruch 4, wobei in Schritt b) die Kristallisation in einem statischen oder dynamischen Zustand durchgeführt wird.

8. Verfahren nach Anspruch 4, wobei in Schritt a) die anfängliche Gelmischung R1/Al₂O₃ = 5,0 ~ 10 aufweist.

9. Verfahren nach Anspruch 4, wobei in Schritt a) die anfängliche Gelmischung R2/Al₂O₃ = 0,25 ~ 1,0 aufweist.

10. Katalysator für die selektive katalytische NOx-Reduktion, der durch Kalzinieren des Molekularsiebs nach einem der Ansprüche 1 bis 3 oder des Molekularsiebs, das nach dem Verfahren nach einem der Ansprüche 3 bis 9 bei 550 bis 700 °C in Luft synthetisiert wurde, erhalten wird.

## Revendications

1. Tamis moléculaire de silicoaluminophosphate (SAPO) contenant du cuivre avec des phases cristallines coexistantes de CHA et GME, dans lequel le diagramme de diffraction des rayons X du tamis moléculaire comprend au moins les pics de diffraction suivants :
| **No.** | **2θ** | **FWHM [2θ] (demi-largeur de pic)** |
|---|---|---|
| 1 | 7,40~7,56 | 0,15~0,35 |
| 2 | 9,20~9,70 | 0,25~0,65 |
| 3 | 12,80~12,92 | 0,06~0,09 |
| 4 | 15,80~16,21 | 0,15~0,40 |
| 5 | 17,60~17,82 | 0,05~0,11 |
| 6 | 20,30~20,90 | 0,15~0,40 |
| 7 | 21,75~22,05 | 0,06~0,09 |
| 8 | 25,65~26,05 | 0,05~0,09 |
| 9 | 30,11~30,96 | 0,20~0,45 |

2. Tamis moléculaire de la revendication 1, dans lequel une structure inorganique du tamis moléculaire a la composition chimique suivante : wCu-(SiₓAl_{y}P_{z})O₂, dans laquelle x, y et z représentent respectivement les fractions molaires de Si, Al et P, leurs plages de fraction molaire sont respectivement x=0,01~0,28, y=0,35~0,55 et z=0,28~0,50 avec x+y+z=1, w est le nombre molaire de Cu par mole de (SiₓAl_{y}P_{z})O₂, et w=0,001~0,124.

3. Tamis moléculaire de la revendication 2, dans lequel la composition chimique anhydre du tamis moléculaire comprenant un agent de matriçage est exprimée par wCu.mR1.nR3.(SiₓAl_{y}P_{z})O₂, dans laquelle R1 est la diisopropanolamine ou la diéthanolamine, R2 est la triméthylamine ; m est le nombre molaire de l'agent de matriçage R1 par mole de (SiₓAl_{y}P_{z})O₂, n est le nombre molaire de l'agent de matriçage R2 par mole de (SiₓAl_{y}P_{z})O₂, m=0,01~0,20, n=0,01~0,10 ; x, y et z représentent respectivement les fractions molaires de Si, Al et P, et leurs plages de fraction molaire sont respectivement x=0,01~0,28, y=0,35~0,55 et z=0,28~0,50 avec x+y+z=1 ; w est le nombre molaire de Cu par mole de (SiₓAl_{y}P_{z})O₂, w = 0,001~0,124.

4. Procédé de synthèse du tamis moléculaire de l'une quelconque des revendications 1 à 3, comprenant les étapes de :
a) mélange d'une source de cuivre, d'eau désionisée, d'agents de matriçage R1 et R2, d'une source de silicium, d'une source d'aluminium et d'une source de phosphore en proportion pour obtenir un mélange de gel initial ayant les rapports molaires suivants :
Cu/Al₂O₃ = 0,01~0,23 ;
SiO₂/Al₂O₃ = 0,03~2,0 ;
P₂O₅/Al₂O₃ = 0,5~1,5 ;
H₂O/Al₂O₃ = 8~40 ;
R1/Al₂Oₛ = 5~20 ;
R2/Al₂O₃ =0,1~1,5 ;
dans lequel R1 est la diisopropanolamine (DIPA) ou la diéthanolamine (DEOA) ; R2 est l'un quelconque de la triméthylamine (TMA), du chlorure de benzyltriméthylammonium (BTACl),de l'hydroxyde de benzyltriméthylammonium (BTAOH) ou d'un mélange de ceux-ci ;
b) introduction du mélange de gel initial obtenu à l'étape a) dans une bouilloire de synthèse haute pression, fermeture de la bouilloire, chauffage jusqu'à 160~220°C et réalisation de la cristallisation pendant 5 à 72 heures ;
et
c) après achèvement de la cristallisation, séparation d'un produit solide, lavage et séchage pour obtenir le tamis moléculaire.

5. Procédé de la revendication 4, dans lequel à l'étape a), un procédé de mélange est le suivant : d'abord le mélange de la source de cuivre avec de l'eau, puis l'ajout de R1 et de R2, l'agitation à température ambiante pendant 0,5~5 heures, puis l'ajout de la source d'aluminium, de la source de silicium et de la source de phosphore à la solution mélangée, et l'agitation du gel mélangé à température ambiante pendant 1-5 heures.

6. Procédé de la revendication 4, dans lequel à l'étape a), la source de silicium est choisie parmi un ou plusieurs des composés suivants : un sol de silice, une silice activée, l'orthosilicate et le métakaolin ; la source d'aluminium est choisie parmi un ou plusieurs des composés suivants : un sel d'aluminium, une alumine activée, la pseudoboehmite, l'alcoxy aluminium et le métakaolin ; la source de phosphore est choisie parmi un ou plusieurs des composés suivants : l'acide orthophosphorique, l'hydrogénophosphate d'ammonium, le dihydrogénophosphate d'ammonium, des composés phosphorés organiques et des oxydes phosphoriques, la source de cuivre est choisie parmi un ou plusieurs des composés suivants : Cu(OAc)₂, CuSO₄, Cu(NO₃)₂ et CuCl₂.

7. Procédé de la revendication 4, dans lequel à l'étape b), la cristallisation est réalisée dans un état statique ou dynamique.

8. Procédé de la revendication 4, dans lequel à l'étape a), le mélange de gel initial a R1/Al₂O₃ = 5,0~10.

9. Procédé de la revendication 4, dans lequel à l'étape a), le mélange de gel initial a R2/Al₂O₃ = 0,25~1,0.

10. Catalyseur pour réduction catalytique sélective de NOₓ, qui est obtenu en calcinant le tamis moléculaire synthétisé selon le procédé de l'une quelconque des revendications 4 à 9 à 550~700°C dans l'air.
